# EUROPEAN PATENT APPLICATION

(11) **EP 3 243 534 A1**
(43) Date of publication of application: **15.11.2017**
(21) Application number: 17169642.0
(22) Date of filing: 05.05.2017
(51) Int. Cl.: A61L 24/00

(54) **AN EMBOLISATION AGENT THAT CAN SEAL OFF VARICOSITY AND/OR HEMORRHOIDS**

(30) Priority: 12.05.2016 TR 201606296
(71) Applicant: INVAMED SAGLIK ILAC SANAYI VE TICARET A.S., Ankara (TR)
(72) Inventor: YILMAZ, MUHAMMET FATIH, ANKARA (TR)
(74) Representative: Karaahmet, Erdogan

(57) **Abstract**

The invention is particularly related to a medical method and medical device used in treatments which enable to obturate, superficial varicosity, Pake and hemorrhoids with embolisation. By means of the device and method subject to the invention the progression of varicose veins, Pake and internal and external hemorrhoids can be stopped, obturated or clogged.

## Description

### Technical Field of the Invention

The invention is particularly related to a medical method and medical device used in treatments which enable to obturate, superficial varicosity, Pake and hemorrhoids with embolisation. By means of the device and method subject to the invention the progression of varicose veins, Pake and internal and external hemorrhoids can be stopped, obturated or obstructed.

### Known State of the Art (Prior Art)

Varicose veins, is a disease that is usually formed on the legs due to the accumulation of blood in the feet for many years as the vein valves has not been functioning sufficiently. Veins that are visible beneath the skin which are twisted, enlarged and swollen are called varicose veins. Hemorrhoids are also formed due to the disfunctioning of the vein system, where the veins at the anus (rectum) area are enlarged, similar to varicosis. The methods that have been mentioned above can also be used for treating hemorrhoids. As these processes are surgical methods, surgical complications may occur due to incisions and suturing. Moreover, following these processes, complications such as bleeding, risks of infection, hematoma, and ecchymosis suture problems may occur.

Embolisation treatment is carried out in order to stop bleeding and to obdurate the veins during the treatment of varicose veins, Pake and hemorrhoids. Embolisation is a method which obstructs the veins in a controlled manner. During embolisation, a small spiral or a chemical agent is delivered through a catheter that has been inserted into the vein that is desired to be obstructed. Embolisation can not only be used to obstruct a vein that is feeding a tumour or a bleeding vein but it can also be used to obdurate or obstruct the veins during a difficult surgical operation. The cyanoacrylates and other embolisation agents known in the market are not used in treating surface Pake procedures and hemorrhoids because they have irritating effects and as they are not flexible. The special concentrated mixture of our invention comprises polymer which is flexible and has the least irritating effect. The hardness of other embolisation agents not only makes it difficult for them to be used in related operations but it also causes intense and common pains in many patients following operations.

Some of the embolisation agents known in the art are as follows:
1. Autologous agents (coagulum, muscle, dura, fat tissue)
2. Particles
   a) Amorphous shaped agents (gel-foam, oxycel, microfiber collagen, polyvinyl alcohol, silk suture)
   b) Spherical shaped agents
      - Absorbed agents (gelatin, albumin, polysaccharides, starch microspheres, ethyl cellulose)
      - Non absorbable agents (glass, wax, silicon, polystyrene, polymethyl methacrylate,)
3. Liquid embolisation agents (fused contrast agent, ethanol, sotradecol, cyanoacrylate)
4. Mechanical embolisation agents
   a) Balloons (balloon occlusion catheters, separable balloons)
   b) Coils (macro coils, micro coils inserted by means of a thruster wire or fluid, mechanical separable coils)

Prior art documents numbered US5758665A and US5611358A have been found to be related to the present invention as they describe a method for treating varicose veins and documents numbered US20110005062A1 and US8936795 B2 have also been found to be related as they describe vascular embolisation.

### Brief Description and Objections of the Invention

The aim of the invention is to inject a very low amount of octabutyl cyanoacrylate (n-butyle and octyl group cyanoacrylate mixture) which is an embolisation agent into the problematic vein using an injector and to apply pressure to the vein for a short period of time and to seal off and obdurate the vein. An embolisation agent whose special concentration and mixtures are determined (n-butyl and octyl group cyanoacrylate mixture) and which has the least irritating effect is obtained by means of the invention.

The patient can be immediately discharged from the hospital following the process of the invention and can return back to his/her normal life. The aim of the invention is to be able to effectively treat such conditions without any pain with outpatient care, without necessitating an incision.

### Detailed Description of the Invention

The embolisation agent subject to the invention is an n-butyl cyano acrylate and octyl cyanoacrylate mixture that is to be applied particularly for varicose veins and hemorrhoids. The n-butyl cyanoacrylate:octyl cyanoacrylate ratios in the mixture have been produced having a ratio of 1:1, 1:2, 1:3, 1:4, 1:5, 1:6, 1:7, 1:8, 1:9, 1:10 and multiples thereof with the CPS (centipoise) ratios of 1-1000 cps.

The embolisation agents used in the invention are materials that can be used in the body in comparison to normal acrylates, that show rapid effects at the area of process and that, can stay soft. Moreover said agent is softer than the acrylates used in other areas of the body and it is rapid acting and it can be delivered by means of an injector. The affects to the surrounding tissue in applications to sensitive areas such as the anal region or applications close to the skin, are reduced by means of the invention, by decreasing the irritating effects of octyl cyanoacrylates and n-butyl cyanoacrylates. Additionally although the n-butyl groups are harder under the skin during sensitive applications, the octyl group remains soft. The n-butyl group makes adhesion faster as it has a higher irritating effect. The aim here, is to provide a mixture having a certain ratio, in order to optimize features such as the irritating effect, adhesion and being able to remain soft.

The storage conditions of the end product are between 6-24°C, and the product is delivered to the user inside standard ampoules.

The embolisation agent which is a mixture of n-butyl cyanoacrylate and octyl cyanoacrylate, has been developed in order to be used during the embolisation process carried out in relation to the treatment of varicose veins and/or hemorrhoids. Moreover it is also possible to use the invention during aneurism embolisation.

An embolisation agent comprising an n-butyl cyanoacrylate, octyl cyanoacrylate and ethyl cyanoacrylate mixture having CPS ratios between 1-1000cps has been developed which shall be used in treating hemorrhoids and surface veins.

## Claims

1. An embolisation agent **characterized in that** it comprises an n-butyl cyanoacrylate and octyl cyanoacrylate mixture having CPS ratios between 1-1000 cps.

2. An embolisation agent **characterized in that** it comprises an n-butyl, cyanoacrylate, and octyl cyanoacrylate and ethyl cyanoacrylate mixture having CPS ratios between 1-1000 cps.

3. An embolisation agent according to claim 1, **characterized in that** the ratio of n-butyl cyanoacrylate: octyl cyanoacrylate is 1:1.

4. An embolisation agent according to claim 1, **characterized in that**, the ratio of n-butyl cyanoacrylate: octyl cyanoacrylate is 1:2.

5. An embolisation agent according to claim 1, **characterized in that**, the ratio of n-butyl cyanoacrylate: octyl cyanoacrylate is 1:3.

6. An embolisation agent according to claim 1, **characterized in that**, the ratio of n-butyl cyanoacrylate: octyl cyanoacrylate is 1:4.

7. An embolisation agent according to claim 1, **characterized in that**, the ratio of n-butyl cyanoacrylate: octyl cyanoacrylate is 1:5.

8. An embolisation agent according to claim 1, **characterized in that**, the ratio of n-butyl cyanoacrylate: octyl cyanoacrylate is 1:6.

9. An embolisation agent according to claim 1, **characterized in that**, the ratio of n-butyl cyanoacrylate: octyl cyanoacrylate is 1:7.

10. An embolisation agent according to claim 1, **characterized in that**, the ratio of n-butyl cyanoacrylate: octyl cyanoacrylate is 1:8.

11. An embolisation agent according to claim 1, **characterized in that**, the ratio of n-butyl cyanoacrylate: octyl cyanoacrylate is 1:9.

12. An embolisation agent according to claim 1, **characterized in that**, the ratio of n-butyl cyanoacrylate: octyl cyanoacrylate is 1:10.

13. Usage of an embolisation agent according to claim 1, during an embolisation process carried out for treating varicose veins and/or hemorrhoids.

14. Usage of an embolisation agent according to claim 1, during an aneurism embolisation.

15. Usage of an embolisation agent according to claim 2, for treating hemorrhoids.

16. Usage of an embolisation agent according to claim 2, for treating surface veins.

17. An embolisation agent according to any of the preceding claims, wherein it comprises dimethylsulfuroxide within the cyanoacrylate solvent.
